# EUROPÄISCHE PATENTANMELDUNG

(11) **EP 1 078 614 A1**
(43) Veröffentlichungstag der Anmeldung: **28.02.2001**
(21) Anmeldenummer: 99810757.7
(22) Anmeldetag: 24.08.1999
(51) Int. Cl.: A61F 5/455

(54) **Einweg -Hilfsvorrichtung zum Urinieren**

(71) Anmelder: Flückiger, Werner, 5727 Oberkulm (CH)
(72) Erfinder: Flückiger, Werner, 5727 Oberkulm (CH)
(74) Vertreter: Heubeck, Bernhard

(57) **Zusammenfassung**

Die Hilfsvorrichtung enthält mindestens ein schlauchartiges Ableitelement (1) mit Wänden (2, 3) aus einem saugfähigen Hygienepapier, welche einen Ableitkanal (4) für Urin begrenzen. Das Ableitelement (1) umfasst einen ersten Endabschnitt (A) mit einer am Körper eines jeweiligen Benutzers positionierbaren Einlasspartie (5) und einen zweiten Endabschnitt (B) mit einer Auslasspartie (6) zum Ableiten des Urins. Der erste Endabschnitt (A) ist durch erste Wandpartien (2a und 3a) gebildet. welche an den Innenseiten mit einer benetzungshemmenden Beschichtung (18) versehen sind. Der zweite Endabschnitt (B) ist durch von benetzungshemmenden Mitteln freie, zweite Wandpartien (2b und 3b) gebildet. Der erste Endabschnitt (A) ist mit einem seitlich abstehenden Griffteil (7) ausgeführt, der durch zungenartige Fortsätze (12) der ersten Wandpartien (2a und 3a) gebildet ist.

Diese Ausführung ermöglicht eine bequeme, hygienisch vorteilhafte Benutzung der Hilfsvorrichtung sowie eine rasche Aufweichung und/oder Auflösung des benutzten Ableitelementes (1) im Wasser einer Toilette.

## Beschreibung

Die Erfindung betrifft eine Einweg- Hilfsvorrichtung zum Urinieren gemäss dem Oberbegriff des Anspruches 1.

Eine aus der US-A-5,722,136 bekannte, für männliche Benutzer bestimmte Hilfsvorrichtung der genannten Art enthält mehrere Ableitelemente, welche je eine hülsenförmige Einlasspartie und eine zum Eintauchen in eine Wasserfüllung einer Toilette bestimmte Auslasspartie aufweisen. Die Ableitelemente sind je aus zwei Papierlagen gebildet, deren Breite annähernd der Breite einer Toilettenpapierrolle entspricht. Die Papierlagen sind an ihren beiden Seitenrändern miteinander verbunden und begrenzen einen Ableitkanal, welcher auf der ganzen Länge mit einer wasserdichten Beschichtung versehen ist.

Die bekannten Ableitelemente weisen je eine relativ grosse Längenabmessung auf, die gegebenenfalls durch Abtrennen eines oder mehrerer Längensegmente an die Körpergrösse des jeweiligen Benutzers angepasst werden soll, um eine direkte Ableitung des Urins in die Wasserfüllung der Toilette zu gewährleisten. Wenn diese Ableitelemente nach deren Benutzung in die Wasserfüllung fallen gelassen werden, besteht die Gefahr, dass die relativ grossflächigen, über ihre ganze Länge beschichteten Papierlagen die Toilette verstopfen, da durch die Beschichtung eine Aufweichung bzw. Auflösung des Papiermaterials verzögert, und damit die störungsfreie Abfuhr der Ableitelemente behindert werden kann.

Der Erfindung liegt die Aufgabe zugrunde, eine insbesondere in dieser Hinsicht verbesserte und einfacher zu handhabende Hilfsvorrichtung der eingangs genannten Art zu schaffen.

Diese Aufgabe wird erfindungsgemäss mit den Merkmalen des Anspruchs 1 gelöst.

Die erfindungsgemäss ausgebildete Hilfsvorrichtung enhält ein gegenüber bisherigen Ausführungen verbessertes Ableitelement in einer einfachen Ausführung, welche eine hygienisch einwandfreie Handhabung bei der Benutzung sowie eine gegenüber bisherigen Ausführungen beschleunigte Aufweichung und/oder Auflösung eines jeweils benutzten Ableitelementes gewährleistet.

Vorteilhafte Ausführungsformen der Erfindung ergeben sich aus den abhängigen Ansprüchen.

Nachstehend wird die Erfindung anhand der beiliegenden Zeichnung erläutert. Es zeigen:
- Fig.1: eine Seitenansicht einer erfindungsgemäss ausgeführten Einweg- Hilfsvorrichtung zum Urinieren für männliche Benutzer,
- Fig.2: die Hilfsvorrichtung nach Fig.1 in einer Draufsicht,
- Fig.3: die Hilfsvorrichtung nach Fig.1 in einer in die Zeichenebene aufgeklappten Innenansicht, und
- Fig.4: eine Seitenansicht einer erfindungsgemässen Hilfsvorrichtung nach einer anderen Ausführungsform.

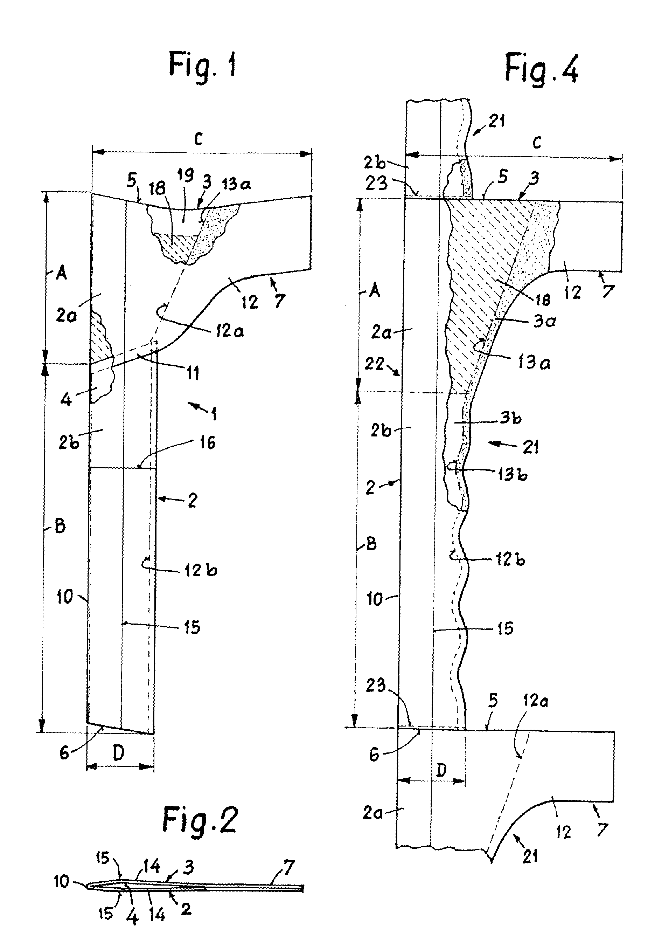

Die Hilfsvorrichtung nach den Fig.1-3 enthält eine Anzahl flexibler, schlauchartiger Ableitelemente 1, von denen nur eines dargestellt ist. Die Ableitelemente 1 dienen zum Erleichtern des Urinierens in einer stehenden Position des jeweiligen Benutzers und zum Ableiten von Urin in eine nicht dargestellte Toilette. Die Hilfsvorrichtung eignet sich insbesondere zur Verwendung in privaten oder anderen Toilettenanlagen, z.B. solchen von Verkehrsmitteln, in denen kein Pissoir vorhanden ist.

Das Ableitelement 1 ist durch zwei flach aneinanderlegbare Wände 2 und 3 aus einem in Wasser aufweich- und/oder zersetzbaren, saugfähigen Hygienepapier gebildet, welche einen von Urin durchströmbaren Ableitkanal 4 begrenzen. Das Ableitelement 1 umfasst einen ersten Endabschnitt A mit einer am Körper des jeweiligen Benutzers ansetzbaren, zur Aufnahme des Penis ausgebildeten Einlasspartie 5, und einen zweiten Endabschnitt B mit einer gegen eine Toilette orientierbaren Auslasspartie 6 zum Ableiten des Urins.

Das Ableitelement 1 ist mit einem am ersten Endabschnitt A ausgebildeten, aus dem Bereich des Ableitkanals 4 seitlich abstehenden Griffteil 7 ausgeführt, der nach Art eines Pistolengriffs geformt ist. Entsprechend wird eine bequeme und vorteilhaft hygienische Handhabung der Hilfsvorrichtung gewährleistet. Gemäss Fig.1 ist der erste Endabschnitt A mit einer Breite C ausgeführt, die z.B. der Breite einer Toilettenpapierrolle entsprechen kann, während der zweite Endabschnitt B eine Breite D aufweist, die einem Bruchteil, darstellungsgemäss etwa einem Drittel der Breite C entspricht. Das Ableitelement 1 besteht daher aus relativ kleinflächigen Abschnitten des Papiermaterials, deren Aufweichung und/oder Auflösung eine vorteilhaft kurze Verweilzeit des benutzten Ableitelementes 1 im Wasser der Toilette erfordert.

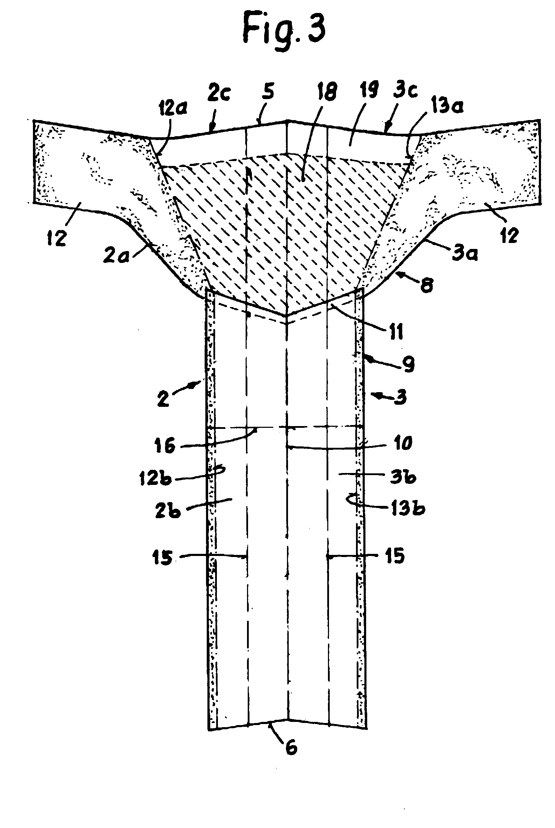

Wie insbesondere aus der Fig.3 hervorgeht, sind die aufgeklappt dargestellten Wände 2 und 3 des Ableitelementes 1 mit einem in dessen Längsrichtung orientierbaren Längsfalz 10 versehen und zu diesem symmetrisch ausgeführt. Die Wände 2 und 3 sind im ersten Endabschnitt A durch zwei erste Wandpartien 2a und 3a, und im zweiten Endabschnitt B durch zwei zweite Wandpartien 2b und 3b gebildet. An den ersten Wandpartien 2a und 3a sind zungenartige Fortsätze 12 ausgeformt, welche je eine Hälfte des Griffteils 7 bilden.

Die Endabschnitte A und B können durch Segmente eines einstückigen Wandteils oder, wie dargestellt, durch Segmente eines ersten Wandteils 8 und eines mit diesem über eine Anschlussstelle 11, z.B. einen Kleberand, verbundenen zweiten Wandteils 9 gebildet sein. Die Wandpartien 2a und 3a bzw. 2b und 3b sind um den Längsfalz 10 faltbar und im Abstand von diesem, zwecks Bildung des Ableitkanals 4, über zusammenführbare seitliche Randbereiche 12a und 13a bzw.12b und 13b miteinander verbindbar, welche in der Zeichnung durch strichpunktierte Begrenzungslinien angedeutet sind.

Die zusammenführbaren Randbereiche können jeweils durch eine mechanische Anordnung, z.B. in Form von ineinandergreifenden Prägungen, oder, wie beim dargestellten Beispiel angenommen, durch ein in Wasser rasch lösbares Klebemittel miteinander verbunden sein. Die Randbereiche 12a und 13a erstrecken sich je über einen der zungenartigen Fortsätze 12 und begrenzen mit dem Längsfalz 10 zwei die Innenseiten des Ableitkanals 4 bildende, über den ersten Endabschnitt A gegen die Einlasspartie 5 divergierend verlaufende Teilsegmente 2c und 3c der Wandpartien 2a und 3a. Die Teilsegmente 2c und 3c sind mit einer kurzzeitig wirksamen, benetzungshemmenden Beschichtung 18 versehen, die in der Zeichnung als gestrichelt schraffierte Fläche dargestellt ist.

Die Beschichtung 18 kann sich im Wesentlichen über den ganzen Endabschnitt A erstrecken oder, wie dargestellt, durch einen im Bereich der Einlasspartie 5 vorgesehenen, von Benetzungsmitteln freien Abstreifrand 19 begrenzt sein. Mit dem entsprechend saugfähig ausgebildeten Abstreifrand 19 kann jeweils, nach der Benutzung des Ableitelementes 1, ein gegebenenfalls vorhandener Urinrest auf hygienisch vorteilhafte Weise abgestreift und entfernt werden.

Die Beschichtung 18 kann durch eine Imprägnierung mit einem umweltfreundlich abbaubaren und bei Berührung mit Wasser rasch auflös- und/oder zersetzbaren Material, z.B. einer Seife, einem Fett oder dgl., gebildet sein. Die Beschichtung 18 kann ferner ein medizinisches Testmittel enthalten, z.B. einen Indikator, der auf die saure oder basische Beschaffenheit des Urins eines jeweiligen Benutzers reagiert. Derart ausgeführte Ableitelemente 1 können daher zugleich als einfache Mittel für entsprechende, z.B. täglich erforderliche medizinische Kontrollfunktionen verwendet werden.

Die Randbereiche 12b und 13b der zweiten Wandpartien 2b und 3b begrenzen im zweiten Endabschnitt B mit dem Längsfalz 10 zwei von benetzungshemmenden Mitteln freie Innenwandpartien des Ableitkanals 4.Der Ableitkanal 4 ist somit nur in dem den Griffteil 7 enthaltenden ersten Endabschnitt A, und damit auf einem Bruchteil, darstellungsgemäss etwa im ersten Drittel, der Länge des Ableitelementes 1 mit dem benetzungshemmenden Material beschichtet, während der Griffteil 7 und die den zweiten Endabschnitt B bildenden Wandpartien 2b und 3b unbeschichtet, und damit beidseitig saugfähig ausgebildet sind. Entsprechend ist der weitaus grösste Teil des Papiermaterials eines jeweils benutzten Ableitelementes 1 in einer vorteilhaft kurzen Zeit im Wasser der Toilette aufweichbar und/oder zersetzbar.

Der in Form eines relativ engen Schlauches ausgebildete zweite Endabschnitt B kann ferner jeweils bereits durch den ihn durchströmenden Urin relativ stark von innen her durchweicht werden. Entsprechend erhöht sich das Eigengewicht des Endabschnitts B, so dass dieser rasch eine senkrechte Ablaufstellung einnimmt und damit ein kontrollierbares gerichtetes Abströmen des Urins gestattet. Der durchtränkte Endabschnitt B bewirkt zugleich eine Bremsung der Strömung des Urins, bevor dieser in einen noch verbleibenden freien Fall übergeht. Dadurch kann das Ableitelement 1 mit einer relativ kleinen, z.B. 40 - 50 cm betragenden Längenabmessung ausgeführt werden, da eine hygienisch einwandfreie Ableitung des Urins bereits erzielt werden kann, wenn die Auslasspartie 6 des Endabschnittes B im Abstand von der Wasserfüllung im oberen Randbereich einer Toilettenschüssel üblicher Bauart positioniert wird.

Bei der beschriebenen Ausführung wird ferner der Zersetzungsprozess des zweiten Endabschnittes B gegenüber demjenigen des ersten Endabschnittes A zeitlich vorverschoben, so dass beim Fallenlassen des gebrauchten Ableitelementes 1 jeweils nur eine entsprechend geringe Menge relativ trockenen Papiermaterials in das Wasser der Toilette gelangt, wodurch eine gegenüber bisherigen Ausführungen wesentlich verbesserte Auflösung des Ableitelementes 1 erzielbar ist.

Wie insbesondere aus der Fig.2 hervorgeht, können die Wände 2 und 3 je mit einer quer zur Längserstreckung des Ableitelementes 1 verlaufenden, zumindest annähernd formstabilen Wölbung 14 ausgeführt sein, wodurch das flach gefaltete Ableitelement 1 mit geringem Aufwand aufgeweitet, und damit dessen Benutzung erleichtert werden kann. Die Wölbungen 14 können darstellungsgemäss durch zwei je über die Wandpartien 2a und 2b bzw. 3a und 3b verlaufende Längsfalze 15 gebildet sein. Das Ableitelement 1 kann ferner mit mindestens einem quer zu dessen Längserstreckung über die Wände 2 und 3 verlaufenden Querfalz 16 versehen und um diesen faltbar ausgeführt sein. Entsprechend ist das Ableitelement 1 in gefaltetem Zustand stapelbar und kann zusammen mit weiteren, je um einen entsprechenden Querfalz 16 gefalteten Ableitelementen 1 in einem nicht dargestellten Vorratspaket untergebracht werden. Im Vorratspaket können die Ableitelemente 1 je mit einem nachfolgenden Ableitelement 1 um den betreffenden Querfalz 16 ineinander faltbar und gegeneinander verhakbar zu einem lose zusammenhängenden Stapel verbunden und einzeln entnehmbar bereitgestellt werden.

In den Figuren 1 und 4 sind einander entsprechende Teile mit gleichen Bezugszeichen versehen. Die Hilfsvorrichtung nach Fig.4, deren Ausführung im Wesentlichen derjenigen nach den Figuren 1-3 entspricht, enthält mehrere flexible, schlauchartige Ableitelemente 21, welche miteinander trennbar verbunden sind, und welche in einem nicht dargestellten Vorratspaket, z.B. auf einer Vorratsrolle, einzeln entnehmbar bereitgestellt werden können. Die Fig.4 zeigt eines der Ableitelemente 21 in einer teilweise aufgebrochenen Vollansicht und zwei benachbarte Ableitelemente 21 in einer Teilansicht. Abweichend von der Ausführung nach den Fig.1-3, sind die ersten und die zweiten Wandpartien 2a und 3a bzw. 2b und 3b jedes Ableitelementes 21 an einem einzigen einstückigen Wandteil 22 ausgebildet, der sich über einen abtrennbaren Längenabschnitt einer einstückigen Bahn aus einem saugfähigen Hygienepapier erstreckt, welche eine Vielzahl solcher Längenabschnitte enthält.

Die ersten Wandpartien 2a und 3a der Ableitelemente 21 sind je über eine im Bereich der Einlasspartie 5 vorgesehene, aufreissbare Anschlussstelle 23, darstellungsgemäss eine gestrichelt angedeutete Perforierung, mit der an den zweiten Wandpartien 2b bzw. 3b des jeweils benachbarten Ableitelementes 21 verbunden. Entsprechend können die auf einer Vorratsrolle aufgewickelten Ableitelemente 21 jeweils einzeln vom nachfolgenden Ableitelement 21 getrennt werden. Es versteht sich, dass auch die Ableitelemente 21 je mit einem entlang der Einlasspartie 5 verlaufenden, saugfähigen Abstreifrand 19 entsprechend der Darstellung nach den Fig.1 und 2 ausgeführt sein können. Die Ableitelemente 21 können auch je mit mindestens einem Querfalz 16 versehen und um diesen faltbar, z.B. in einem nicht dargestellten Behälter, einzeln entnehmbar gestapelt werden.

Die Randbereiche 12b und 13b der zweiten Wandpartien 2b bzw. 3b können darstellungsgemäss entlang einer zumindest annähernd wellenförmig ausgebildeten Anschlussstelle zu einer entsprechend profilierten Wandung des zweiten Endabschnitts B verbunden sein. Dadurch kann die im Endabschnitt B erzielbare Bremsung des abzuleitenden Urins verstärkt und zugleich eine raschere Durchtränkung des zweiten Endabschnitt B erreicht werden.

Nach einer weiteren, nicht dargestellten Ausführungsform können die Ableitelemente 1 und/oder 21 auch je durch zweilagig ausgeführte, an den beiden Seitenrändern miteinander verbundene Wände gebildet sein. Ferner kann auch das Ableitelement 1 über Anschlussstellen 23 mit benachbarten Ableitelementen 1 verbunden sein.

## Patentansprüche

1. Einweg- Hilfsvorrichtung zum Urinieren, mit mindestens einem schlauchartigen Ableitelement (1; 21), welches durch flach aneinanderlegbare Wände (2, 3) aus einem in Wasser aufweich- und/oder zersetzbaren Material gebildet ist, die einen mit einer benetzungshemmenden Beschichtung (18) versehenen Ableitkanal (4) begrenzen, und welches einen ersten Endabschnitt (A) mit einer am Körper eines jeweiligen Benutzers positionierbaren Einlasspartie (5), und einen zweiten Endabschnitt (B) mit einer Auslasspartie (6) zum Ableiten von Urin in eine Toilette oder dgl. aufweist, dadurch gekennzeichnet, dass das Ableitelement (1; 21) mit einem am ersten Endabschnitt (A) seitlich abstehend angeordneten Griffteil (7) ausgeführt ist, dass die Beschichtung (18) an den Innenseiten von den ersten Endabschnitt (A) bildenden, ersten Wandpartien (2a und 3a) angebracht ist, und dass der zweite Endabschnitt (B) durch von benetzungshemmenden Mitteln freie, zweite Wandpartien (2b und 3b) gebildet ist.

2. Hilfsvorrichtung nach Anspruch 1, dadurch gekennzeichnet, dass die ersten Wandpartien (2a und 3a) mit einem entlang der Einlasspartie (5) verlaufenden, von benetzungshemmenden Mitteln freien Abstreifrand (19) ausgeführt sind.

3. Hilfsvorrichtung nach Anspruch 1 oder 2, dadurch gekennzeichnet, dass die ersten und die zweiten Wandpartien (2a und 3a bzw. 2b und 3b) aus einem saugfähigen Hygienepapier gebildet sind.

4. Hilfsvorrichtung nach einem der vorangehenden Ansprüche, dadurch gekennzeichnet, dass der Griffteil (7) in Form eines Pistolengriffs ausgeführt ist, der durch zungenartige Fortsätze (12) der ersten Wandpartien (2a und 3a) gebildet ist.

5. Hilfsvorrichtung nach einem der vorangehenden Ansprüche, dadurch gekennzeichnet, dass die Wandpartien (2a und 3a bzw. 2b und 3b) durch Segmente mindestens eines einstückigen Wandteils (7, 8; 22) gebildet sind, welche um einen in Längsrichtung des Ableitelementes (1; 21) orientierbaren Längsfalz (10) faltbar ausgeführt und über je im Abstand von diesem verlaufende Randbereiche (12a und 13a bzw. 12b und 13b) des Wandteils (7, 8; 22) miteinander verbunden sind.

6. Hilfsvorrichtung nach Anspruch 5, dadurch gekennzeichnet, dass die Randbereiche (12b und 13b) der zweiten Wandpartien (2b und 3b) entlang einer zumindest annähernd wellenförmig ausgebildeten Anschlussstelle miteinander zu einem entsprechend profilierten Wandungsabschnitt des Ableitelementes (21) verbunden sind.

7. Hilfsvorrichtung nach einem der vorangehenden Ansprüche, dadurch gekennzeichnet, dass die ersten und die zweiten Wandpartien (2a und 3a bzw. 2b und 3b) je mit einer quer zur Längserstreckung des Ableitelementes (1; 21) verlaufenden, zumindest annähernd formstabilen Wölbung (14) ausgeführt sind.

8. Hilfsvorrichtung nach einem der vorangehenden Ansprüche, dadurch gekennzeichnet, dass die Beschichtung (18) der ersten Wandpartien (2a und 3a) ein auf eine bestimmte Beschaffenheit des Urins des jeweiligen Benutzers reagierendes medizinisches Testmittel enthält.
